# EUROPÄISCHE PATENTSCHRIFT

(11) **EP 1 139 925 B1**
(45) Veröffentlichungstag und Bekanntmachung des Hinweises auf die Patenterteilung: **01.09.2004**
(21) Anmeldenummer: 99950424.4
(22) Anmeldetag: 03.11.1999
(51) Int. Cl.: A61F 2/34

(54) **KÜNSTLICHE HÜFTGELENKPFANNE**
ARTIFICIAL HIP JOINT SOCKET
CAVITE COTYLOIDE ARTIFICIELLE

(43) Veröffentlichungstag der Anmeldung: 10.10.2001
(73) Patentinhaber: Biomet Merck GmbH, 3216 Ried b. Kerzers (CH)
(72) Erfinder: KOCH, Rudolf, CH-4436 Oberdorf (CH)
(74) Vertreter: Lusuardi, Werther Giovanni, Dr.
(86) Internationale Anmeldenummer: PCT/CH1999/000514
(87) Internationale Veröffentlichungsnummer: WO 2001/032108

(56) Entgegenhaltungen:
- DE-A- 19 701 778
- FR-A- 2 700 686
- US-A- 3 818 512
- US-A- 3 894 297
- US-A- 5 609 648

## Beschreibung

Die Erfindung betrifft eine künstliche Hüftgelenkpfanne, gemäss dem Oberbegriff des Patentanspruchs 1.

Solche Hüftgelenkpfannen eignen sich insbesondere für die zementfreie Implantation in das Acetabulum.

Aus dem Stand der Technik ist eine derartige Hüftgelenkpfanne bekannt, bei welcher die verschleissfeste Innenschale einen zylindrischen Aufsatz mit einem darauf angeordneten Längskamm aufweist und die Polyäthylenzwischenschale eine entsprechende zylindrische Vertiefung mit zusätzlichem Langloch an ihrem Grund aufweist. Der zylindrische Aufsatz und die entsprechende Vertiefung dienen der Führung der beiden Schalen und der in das Längsloch passende Längskamm dient der Rotationssicherung zwischen den beiden Schalen. Dieses vormontierte System kann intraoperativ mit einer äusseren Metallschale lösbar verbunden werden. Dies geschieht über eine deformierbare Polyäthylen-Schnappvorrichtung. Die Aussenschale aus Titan oder Titanlegierung ist zementfrei mit dem Knochen verbunden.

Nachteilig bei dieser bekannten Hüftgelenkpfanne ist die grosse Bauhöhe der verschleissfesten Innenschale, welche sich durch die Superposition der Führungsund Rotationssicherungs-Mittel ergibt. Ein weiterer Nachteil besteht darin, dass der Längskamm der Innenschale im Längsloch der Zwischenschale auf Grund seiner Geometrie Spitzenbeanspruchungen verursacht. Dies verhindert auch den Einsatz von keramischen Materialien, welche auf solche Beanspruchungen ungünstig reagieren.

Aus der US-A-3 818 512 SHERSHER ist ein Hüftprothese bekannt, welche zwischen dem Kugelkopf des Schaftes und der künstlichen Hüftpfanne ein separates Zwischenstück aufweist, so dass die Hüftschale zweiteilig ausgebildet ist.

Aus der FR-A-2 700 686 VOYDEVILLE ist auch eine dreiteilige Hüftschale bekannt, bei welcher allerdings die Aussenschale und Zwischenschate nicht gegen Rotation gesichert ist. Dieses Dokument stellt den nächstkommenden Stand der Technik dar.

Hier will die Erfindung Abhilfe schaffen. Der Erfindung liegt die Aufgabe zugrunde, eine künstliche Hüftgelenkpfanne zu schaffen, welche bei einer minimalen Bauhöhe eine optimale Verteilung der Auflagekräfte bewirkt.

Zur Lösung dieses Problems ist die eingangs genannte Anordnung durch die Merkmale des kennzeichnenden Teils des unabhängigen Anspruchs 1 weitergebildet.

Damit ist der Vorteil erzielbar, dass die prismatisch ausgebildeten Mittel an der Innen- und Zwischenschale, diese gleichzeitig führen als auch gegen Rotation und Kippen sichern, so dass insgesamt eine reduzierte Bauhöhe resultiert. Die reduzierte Bauhöhe lässt einerseits gesamthaft kleinere Pfannen mit dünneren Wandstärken der Zwischenschale zu, anderseits ist es möglich auch bei sehr kleinen Pfannen eine Kongruenz des Radius-Einsteckpunktes von Innen-Gleitfläche und Aussengeometrie zu erzielen. Dies entspricht einer kinematisch optimalen Rekonstruktion des Hüftgelenkes.

Gegenüber dem Stand der Technik ergibt sich eine bessere und in jeder Richtung gleichmässigere Verteilung der Kipp- und Rotationskräfte zwischen Innen- und Zwischenschale, d.h. von der Gelenkkugel auf die Innenschale wirkende Momente werden optimal (via Zwischen- und Aussenschale) auf den Beckenknochen übertragen.

Bei einer bevorzugten Ausführungsform stellt der Aufsatz der Innenschale und die entsprechende Vertiefung in der Zwischenschale im wesentlichen ein reguläres vierseitiges Prisma dar. Damit wird eine optimale Verteilung der Auflage kräfte erreicht. Die Länge der regelmässigen Seiten des vierseitigen Prismas können im Bereich von 10 bis 18 mm, vorzugsweise von 13 bis 15 mm liegen.

Vorteilhafterweise sind die prismatischen Längskanten des Aufsatzes abgerundet oder abgeschnitten, wobei die Längskanten typischerweise auf einer Länge von 0,8 mm bis 1,5 mm abgeschnitten sind. Die durch die Abrundung, bzw. Abkantung erzielten Facetten bedeuten eine weitere Einsparung von Wand stärke an der Zwischenschale, was den Einbau des Systems auch in kleine Aussenschalen und damit in kleine Acetabuli ermöglicht.

Die Höhe des prismatischen Aufsatzes und der prismatischen Vertiefung sollte im Bereich von 2 bis 6 mm, vorzugsweise von 3,5 bis 4,5 mm liegen.

Die Gesamthöhe der Innenschale, einschliesslich des Aufsatzes sollte höchstens 65 %, vorzugsweise höchstens 60 % des maximalen Durchmessers der Innenschale (3) betragen.

Vorzugsweise besteht die verschleissfeste Innenschale aus einer CoCr-Legierung oder aus Keramik (z.B. aus Al₂O₃).

Die Erfindung und Weiterbildungen der Erfindung werden im folgenden anhand der teilweise schematischen Darstellung eines Ausführungsbeispiels noch näher erläutert.

Es zeigen:
Fig. 1 eine perspektivische Ansicht der Innenschale der erfindungsgemässen Hüftgelenkpfanne;
Fig. 2 eine Seitenansicht der Innenschale gemäss Fig. 1;
Fig. 3 eine Aufsicht auf die Innenschale gemäss Fig. 1; und
Fig. 4 einen Querschnitt durch Aussenschale und Zwischenschale der erfindungsgemässen Hüftgelenkpfanne.

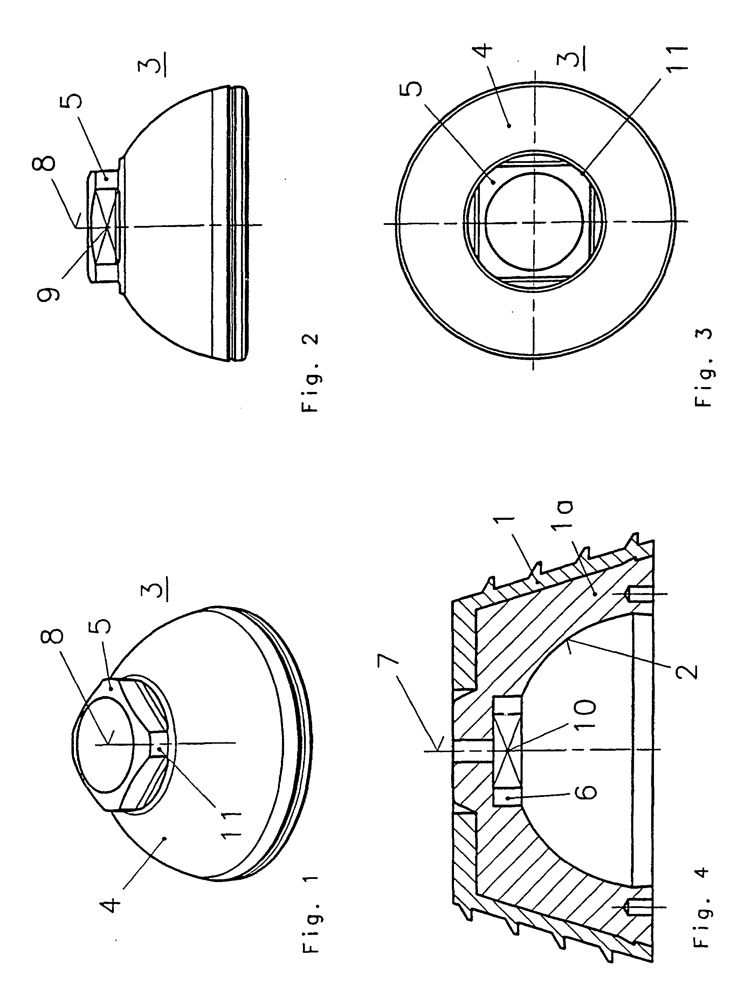

Die in den Fig. 1 bis 4 dargestellte künstliche Hüftgelenkpfanne umfasst einerseits eine zur Verankerung im Beckenknochen bestimmte Aussenschale 1 aus Titan mit Zentralachse 7, welche in lösbarer Weise formschlüssig mit einer Kunststoff-Zwischenschale 1a mit hohlkugelförmiger Innenseite 2 verbindbar ist; anderseits eine zur Aufnahme einer Gelenkkugel eines Femurschafts bestimmte verschleissfeste Innenschale 3 aus Aluminiumoxidkeramik mit einer sphärischen Aussenseite 4 und einer Zentralachse 8.

Die Aussenseite 4 der Innenschale 3 ist in nicht-lösbarer Weise formschlüssig an die Innenseite 2 der Zwischenschale 1a zur Anlage bringbar, so dass die beiden Zentralachsen 7,8 übereinstimmen.

An der Innen- und Zwischenschale 3,1a sind ineinandergreifende Mittel vorgesehen, welche die zur Anlage gebrachten Innenund Zwischenschalen 3,1a gegen Rotation um ihre Zentralachsen 7,8 und gegen Kippung relativ zueinander sichern. Diese Anti-Rotations-Mittel und Anti-Kipp-Mittel bestehen einerseits aus einem am Scheitelpunkt 9 der Innenschale 3 auf der Aussenseite 4 angebrachten prismatischen Aufsatz 5 und anderseits aus einer am Scheitelpunkt 10 der Zwischenschale 1a auf der Innenseite 2 angebrachte prismatische Vertiefung 6. Der Aufsatz 5 und die Vertiefung 6 sind im wesentlichen kongruent und symmetrisch zu den Zentralachsen 7,8 angeordnet und stellen ein reguläres vierseitiges Prisma dar. Die Länge der regelmässigen Seiten des vierseitigen Prismas beträgt 14 mm; sie sind jedoch auf einer Länge von 1,2 mm abgeschnitten, so dass vier Facetten 11 resultieren (Fig. 1).

Die Höhe des prismatischen Aufsatzes 5 und der prismatischen Vertiefung 6 beträgt 4 mm. Die Gesamthöhe der Innenschale 3, einschliesslich des Aufsatzes 5 beträgt 55 % des maximalen Durchmessers der Innenschale 3.

Im folgenden werden die einzelnen Schritte zur Implantation der erfindungsgemässen, künstlichen Hüftgelenkpfanne beschrieben:
1. Auffräsen des Acetabulums (sphärisch oder konisch).
2. Einbringen der zementfrei verankerbaren Aussenschale 1 aus Titan (sphärisch = "pressfit" oder konisch = Schraubenpfanne) in das gemäss Schritt 1 vorbereitete Acetabulum.
3. Allenfalls Setzen von Knochenschrauben durch geeignete Löcher in der modularen Aussenschale 1 aus Titan.
4.1. Einschlagen des Sandwich-Inlays in die Aussenschale 1, wobei das Sandwich-Inlay aus der Kunststoff-Zwischenschale 1a und der verschleissfesten Innenschale 3 besteht.
4.2. Die Verbindung zwischen der Kunststoff-Zwischenschale 1a und der Aussenschale 1 erfolgt über einen zeichnerisch nicht dargestellten, konventionellen Schnappmechanismus aus Polyethylen.
4.3. Die Verankerung der verschleissfesten Innenschale 3 mit der Kunststoff-Zwischenschale 1a geschieht mittels des erfindungsgemässen prismatischen Aufsatzes 5 und der dazu korrespondierenden prismatischen Vertiefung 6 und einem konventionellen Schnappverschluss, bzw. einem Gegenkonus.
5. Reposition des Hüftgelenkes mit Einschnappen der Gelenkkugel in die künstliche Hüftpfanne.

## Patentansprüche

1. Künstliche Hüftgelenkpfanne mit
A) einer zur Verankerung im Beckenknochen bestimmten Aussenschale (1) mit Zentralachse (7), welche in lösbarer Weise formschlüssig mit einer Kunststoff-Zwischenschale (1a) mit hohlkugelförmiger Innenseite (2) verbindbar ist;
B) einer zur Aufnahme einer Gelenkkugel eines Femurschafts bestimmten, verschleissfesten Innenschale (3) mit einer sphärischen Aussenseite (4) und einer Zentralachse (8), wobei
C) die Aussenseite (4) der Innenschale (3) in nicht-lösbarer Weise formschlüssig an die Innenseite (2) der Zwischenschale (1a) zur Anlage bringbar ist, so dass die beiden Zentralachsen (7,8) übereinstimmen; und
D) an der Innen- und Zwischenschale (3,1a) ineinandergreifende Mittel vorgesehen sind, welche die zur Anlage gebrachten Innen- und Zwischenschalen (3,1 a) gegen Rotation um ihre Zentralachsen (7,8) und gegen Kippung relativ zueinander sichern,
**dadurch gekennzeichnet, dass**
die Mittel zur Rotations- und Kipp-Sicherung einerseits aus
E) einem am Scheitelpunkt (9) der Innenschale (3) auf der Aussenseite (4) angebrachten prismatischen Aufsatz (5); und anderseits aus
F) einer am Scheitelpunkt (10) der Zwischenschale (1a) auf der Innenseite (2) angebrachten prismatischen Vertiefung (6), bestehen, wobei
G) Aufsatz (5) und Vertiefung (6) im wesentlichen kongruent sind;
H) Aufsatz (5) und Vertiefung (6) im wesentlichen symmetrisch zu den Zentralachsen (7,8) angeordnet sind;
I) die Höhe des prismatischen Aufsatzes (5) und der prismatischen Vertiefung (6) im Bereich von 2 bis 6 mm, liegt; und
K) die Gesamthöhe der Innenschale (3), einschliesslich des Aufsatzes (5) höchstens 65 % des maximalen Durchmessers der Innenschale (3) beträgt.

2. Hüftgelenkpfanne nach Anspruch 1, **dadurch gekennzeichnet, dass** Aufsatz (5) und Vertiefung (6) im wesentlichen ein reguläres vierseitiges Prisma darstellen.

3. Hüftgelenkpfanne nach Anspruch 2, **dadurch gekennzeichnet, dass** die Länge der regelmässigen Seiten des vierseitigen Prismas im Bereich von 10 bis 18 mm, vorzugsweise 13 bis 15 mm liegt.

4. Hüftgelenkpfanne nach einem der Ansprüche 1 bis 3, **dadurch gekennzeichnet, dass** mindestens die prismatischen Längskanten (11) des Aufsatzes (5) abgerundet oder abgeschnitten sind.

5. Hüftgelenkpfanne nach Anspruch 4, **dadurch gekennzeichnet, dass** die Längskanten auf einer Länge von 0,8 mm bis 1,5 mm abgeschnitten sind.

6. Hüftgelenkpfanne nach einem der Ansprüche 1 bis 5, **dadurch gekennzeichnet, dass** die Höhe des prismatischen Aufsatzes (5) und der prismatischen Vertiefung (6) im Bereich von 3,5 bis 4,5 mm liegt.

7. Hüftgelenkpfanne nach einem der Ansprüche 1 bis 6, **dadurch gekennzeichnet, dass** die Gesamthöhe der Innenschale (3), einschliesslich des Aufsatzes (5) 60 % des maximalen Durchmessers der Innenschale (3) beträgt.

8. Hüftgelenkpfanne nach Anspruch 7, **dadurch gekennzeichnet, dass** die Gesamthöhe der Innenschale (3), einschliesslich des Aufsatzes (5) höchstens 55 % des maximalen Durchmessers der Innenschale (3) beträgt.

9. Hüftgelenkpfanne nach einem der Ansprüche 1 bis 8, **dadurch gekennzeichnet, dass** die verschleissfeste Innenschale (3) aus CoCr-Legierung oder aus Keramik besteht.

10. Hüftgelenkpfanne nach Anspruch 9, **dadurch gekennzeichnet, dass** die verschleissfeste Innenschale (3) aus Al₂O₃ besteht.

## Claims

1. An artificial hip-joint socket comprising
(a) an outer shell (1) comprising a central axis (7) to be anchored into the hip bone and which can be detachably affixed in geometrically locking manner to a plastic intermediate shell (1a) comprising a hollow-spherical inside surface (2),
(b) a wear-resistant inner shell (3) comprising a spherical outer side (4) and a central axis (8) designed to receive a joint ball of a femur shaft, where
(c) the outer side (4) of the inner shell (3) can be made to undetachably rest in geometrically locking manner on the inner side (2) of the intermediate shell (1a) in such manner that the two central axes (7, 8) shall coincide, and
(d) mutually engaging elements are present at the inner and outer shells (3 and 1a resp.) which secure the mutually contacting inner and intermediate shells (3 and 1a resp.) against rotation about their resp. central axes (7 and 8) and against mutual canting,
**characterized in that**
the elements precluding rotation and canting resp. consist of
(e) a prismatic salient (5) configured at a vertex (9) of the inner shell (3) on the outer side (4), and
(f) a prismatic recess (6) configured at the vertex (10) of the intermediate shell (1a) on the inner side (2), where
(g) the salient (5) and the recess (6) are configured in substantially congruent manner,
(h) the salient (5) and the recess (6) are configured substantially symmetrically to the central axes (7 resp. 8) , and
(i) The elevation of the prismatic salient (5) and the elevation of the prismatic recess (6) each are in the range of 2 to 6 mm; and
(k) the total elevation of the inner shell (3) inclusive the elevation (5) amounts at most to 65 % of the maximum diameter of the inner shell (3).

2. Hip-joint socket as claimed in claim 1, **characterized in that** the salient (5) and the recess (6) substantially each represent a regular tetragonal prism.

3. Hip-joint socket as claimed in claim 2, **characterized in that** the length of the regular sides of the tetragonal prism is in the range of 10 to 18 mm, preferably 13 to 15 mm.

4. Hip-joint socket as claimed in one of claims 1 through 3, **characterized in that** at least the prismatic longitudinal edges (11) of the salient (5) are rounded or cut off.

5. Hip-joint socket as claimed in claim 4, **characterized in that** the longitudinal edges are cut off by a length of 0.8 to 1.5 mm.

6. Hip-joint socket as claimed in one of claims 1 through 5, **characterized in that** the elevation of the prismatic salient (5) and that of the prismatic recess (6) shall be each in the range of 3.5 to 4.5 mm.

7. Hip-joint socket as claimed in one of claims 1 through 6, **characterized in that** the total elevation of the inner shell (3) inclusive the elevation (5) amounts at most to 60 % of the maximum diameter of the inner shell (3).

8. Hip-joint socket as claimed in claim 7, **characterized in that** the total height of the inner shell (3), inclusive the salient (5), at most amounts to 55 % of the maximum diameter of the inner shell (3).

9. Hip-joint socket as claimed in one of claims 1 through 8, **characterized in that** the wear-resistant inner shell (3) is composed of a CoCr alloy or of ceramics.

10. Hip-joint socket as claimed in claim 9, **characterized in that** the wear-resistant inner shell (3) is made of Al₂O₃ .

## Revendications

1. Cavité articulaire artificielle de la hanche comprenant
A) une coque extérieure (1) avec un axe central (7) destinée à être ancrée dans l'os du bassin, laquelle peut être reliée de manière amovible, par conjugaison de forme, à une coque intermédiaire (la) en matière plastique à face intérieure (2) en forme de boule creuse;
B) une coque intérieure (3) résistante à l'usure avec une face extérieure sphérique (4) et un axe central (8), laquelle est destinée à recevoir une sphère articulaire d'une tige fémorale,
C) la face extérieure (4) de la coque intérieure (3) pouvant être placée de manière non-amovible, par conjugaison de forme, contre la face intérieure (2) de la coque intermédiaire (la), de sorte que les deux axes centraux (7,8) coïncident; et
D) sur les coques intérieure et intermédiaire (3,1a) étant prévus des moyens s'engrenant l'un dans l'autre, qui empêchent les coques intérieure et intermédiaire (3,1a) disposées l'une contre l'autre de tourner sur leurs axes centraux (7,8) et de basculer l'une par rapport à l'autre,
**caractérisée en ce que**
les moyens anti-rotation et anti-basculement sont composés, d'une part,
E) d'un élément rapporté prismatique (5) disposé au sommet (9) de la coque intérieure (3), sur la face extérieure (4) de celle-ci; et, d'autre part,
F) d'un creux prismatique (6) disposé au sommet (10) de la coque intermédiaire (la), sur la face intérieure (2) de celle-ci,
G) l'élément rapporté (5) et le creux (6) étant essentiellement congruents;
H) l'élément rapporté (5) et le creux (6) étant disposé essentiellement de manière symétrique aux axes centraux (7,8);
I) la hauteur de l'élément rapporté prismatique (5) et du creux prismatique (6) étant comprise entre 2 et 6 mm; et
K) la hauteur totale de la coque intérieure (3), élément rapporté (5) y compris, constituant tout au plus 65 % du diamètre maximal de la coque intérieure (3).

2. Cavité articulaire de la hanche selon la revendication 1, **caractérisée en ce que** l'élément rapporté (5) et le creux (6) constituent essentiellement un prisme régulier à quatre côtés.

3. Cavité articulaire de la hanche selon la revendication 2, **caractérisée en ce que** la longueur des côtés réguliers du prisme quadrilatère est comprise entre 10 et 18 mm, de préférence entre 13 et 15 mm.

4. Cavité articulaire de la hanche selon l'une des revendications 1 à 3, **caractérisée en ce qu'**au moins les arêtes longitudinales prismatiques (11) de l'élément rapporté (5) sont arrondies ou coupées.

5. Cavité articulaire de la hanche selon la revendication 4, **caractérisée en ce que** les arêtes longitudinales sont coupées à une longueur comprise entre 0,8 mm et 1,5 mm.

6. Cavité articulaire de la hanche selon l'une des revendications 1 à 5, **caractérisée en ce que** la hauteur de l'élément rapporté prismatique (5) et du creux prismatique (6) est comprise entre 3,5 et 4,5 mm.

7. Cavité articulaire de la hanche selon l'une des revendications 1 à 6, **caractérisée en ce que** la hauteur totale de la coque intérieure (3), élément rapporté (5) y compris, constitue 60 % du diamètre maximal de la coque intérieure (3).

8. Cavité articulaire de la hanche selon la revendication 7, **caractérisée en ce que** la hauteur totale de la coque intérieure (3), élément rapporté (5) y compris, constitue tout au plus 55 % du diamètre maximal de la coque intérieure (3).

9. Cavité articulaire de la hanche selon l'une des revendications 1 à 8, **caractérisée en ce que** la coque intérieure (3) résistante à l'usure est réalisée en alliage de CoCr ou en céramique.

10. Cavité articulaire de la hanche selon la revendication 9, **caractérisée en ce que** la coque intérieure (3) résistante à l'usure est réalisée en Al₂O₃.
